# EUROPEAN PATENT APPLICATION

(11) **EP 2 998 739 A1**
(43) Date of publication of application: **23.03.2016**
(21) Application number: 15182919.9
(22) Date of filing: 28.08.2015
(51) Int. Cl.: G01N 33/50, G01N 33/20, G01N 21/64, G01N 33/52

(54) **ZINC FLUORESCENT PROBE**

(30) Priority: 16.09.2014 WO PCT/CN2014/086600
(71) Applicant: The Procter & Gamble Company, Cincinnati, OH 45202 (US)
(72) Inventor: SHI, Yunming, 101312 Beijing (CN); STRAND, Ross, 138638 Singapore (SG); YI, Tao, 200433 Shanghai (CN); LAN, Haichuang, 200433 Shanghai (CN); WEN, Ying, 200433 Shanghai (CN)
(74) Representative: Joos, Uta Susanne

(57) **Abstract**

Rhodamine B derivative selectively chelates Zn²⁺ to act as a fluorescent probe.

## Description

### FIELD OF THE INVENTION

Fluorescent probes selectively detecting zinc (Zn²⁺).

### BACKGROUND OF THE INVENTION

Zinc (Zn²⁺) is used widely in personal care products and house hold products as a chemical adjunct for many purposes including the inhibition of bacterial growth, mitigating halitosis, and providing anti-dandruff benefits. Furthermore, zinc is the second most abundant transition metal ion in the human body, and is an essential cofactor in many biological processes such as brain function and pathology, gene transcription, immune function, and mammalian reproduction. Given this wide spread use of zinc and importance of zinc in biological systems, there is an ongoing need to develop methods to detect zinc in various systems to further study zinc.

Rhodamine B is widely used as a fluorescent probe for the detection metal ion and biomolecule, due to its high quantum yields, water solubility and good photostability.

There is a need for chemical probe that is highly selective for Zn²⁺ in the presence of various metal ions and will exhibit high fluorescence upon Zn²⁺ chelation. There is a further need for such probe for use in living cells.

It is an advantage to have a probe that is relatively easy and simple to synthesize.

It is a further advantage to have a probe that demonstrates good solubility range (polar and non-polar).

It is yet a further advantage to have a probe for Zn²⁺ imaging in living prokaryotic and/or eukaryotic systems.

Rhodamine-based, thiopheneyl-containing, fluorogenic chemosensor for Fe³⁺ has been reported. Lijun Tang, et al., "A new dual chromo- and fluorogenic chemosensor for Fe3+ in aqueous solution," J. Chemical Research, (April 2010), 216-218.

Rhodamine-based, pyridinyl-containing, fluorescent chemosensor for detection of citrate and Pb²⁺ in aqueous solution. Chun-Yan Li, et al., "Colorimetric and fluorescent chemosensor for citrate based on a rhodamine and Pb2+ complex in aqueous solution," Analytica Chimica Acta, 774 (2013) 79-84.

### SUMMARY OF THE INVENTION

The present invention addresses this need by the surprising discovery of a fluorescent Zn²⁺ probe containing rhodamine B derivative moiety as fluorophore, linked via amide moiety to 2-methyl-1H-pyrrole, which can selectively chelate Zn²⁺. The use of this class of probe compounds is demonstrated as an imaging probe for monitoring Zn²⁺ in living cells to study the physiological function of Zn²⁺ in biological systems. Alternatively, this class of probe can be used to assess for the presence of Hg²⁺.

A first aspect of the invention provides for a compound having the following Formula (I): 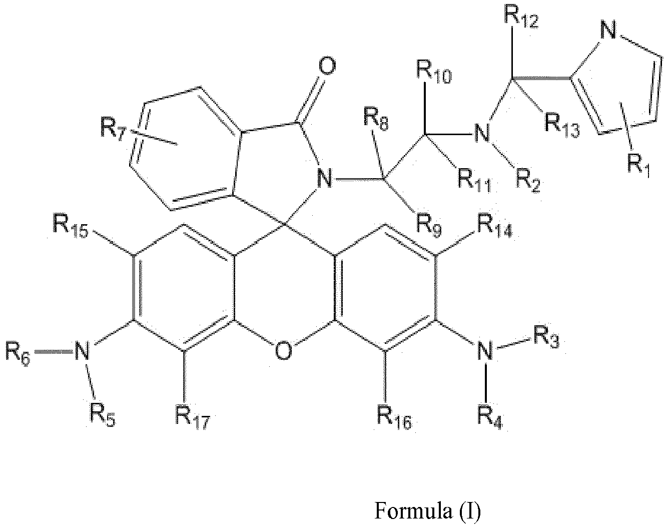 wherein R₁, R₂, R₃, R₄, R₅, R₆, R₇, R₅, R₉, R₁₀, R₁₁, R₁₂, R₁₃, R₁₄, R₁₅, R₁₆, and R₁₇ are each independently a hydrogen or a hydrocarbyl; or an optical isomer, diastereomer or enantiomer of Formula (I), or a salt thereof.

In a preferred embodiment, the compound is a fluorescent probe having a Formula (I) wherein:
R₁, R₂, R₃, R₄, R₅, R₆, R₇, R₈, R₉, R₁₀, R₁₁, R₁₂, R₁₃, R₁₄, R₁₅, R₁₆, and R₁₇ are each independently selected from the group consisting of hydrogen, alkyl, alkenyl, alkynyl, cycloalkyl, cycloalkenyl, aryl, heteroakyl, heteroalkenyl, heteroalkynyl, heterocycloalkyl, heterocycloalkenyl, and heteroaryl;
or wherein R₆ and R₁₅; R₅ and R₁₇; or R₅ and R₆; may together form a moiety selected from the group consisting of a cycloalkyl, cycloalkenyl, aryl, heterocycloalkyl, heterocycloalkenyl, and heteroaryl;
or wherein R₄ and R₁₆; R₃ and R₁₄; or R₃ and R₄ may together form a moiety selected from the group consisting of a cycloalkyl, cycloalkenyl, aryl, heterocycloalkyl, heterocycloalkenyl, and heteroaryl; and
wherein anyone of the aforementioned maybe substituted or unsubstituted.

Another aspect of the invention provides a compound of Formula (II): 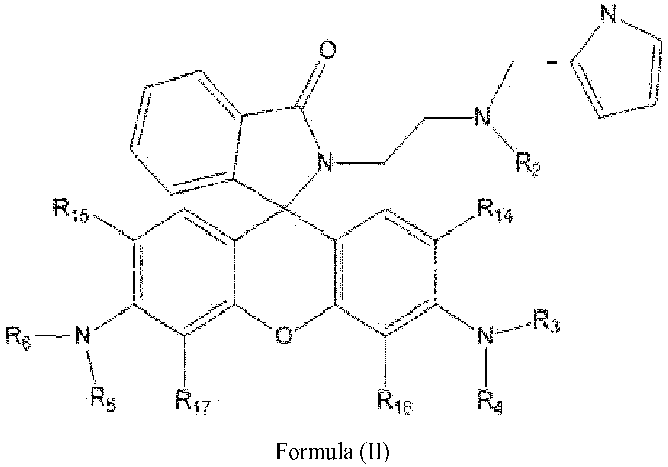 wherein R₂, R₃, R₄, R₅, R₆, R₁₄, R₁₅, R₁₆, and R₁₇ are each independently selected from the group consisting of hydrogen, alkyl, alkenyl, alkynyl, cycloalkyl, cycloalkenyl, aryl, heteroakyl, heteroalkenyl, heteroalkynyl, heterocycloalkyl, heterocycloalkenyl, and heteroaryl;
or wherein R₆ and R₁₅; R₅ and R₁₇; or R₅ and R₆; may together form a moiety selected from the group consisting of a cycloalkyl, cycloalkenyl, aryl, heterocycloalkyl, heterocycloalkenyl, and heteroaryl;
or wherein R₄ and R₁₆; R₃ and R₁₄; or R₃ and R₄ may together form a moiety selected from the group consisting of a cycloalkyl, cycloalkenyl, aryl, heterocycloalkyl, heterocycloalkenyl, and heteroaryl; and
wherein anyone of the aforementioned maybe substituted or unsubstituted.

In another aspect of the invention, a compound according to Formula (I) is provided, wherein the compound is selected from the group consisting of:
(a) 2-(2-(((1*H-*pyrrol-2-yl)methyl)amino)ethyl)-3',6'-diaminospiro[isoindoline-1,9'-xanthen]-3-one;
(b) 2-(2-(((1H-pyrrol-2-yl)methyl)amino)ethyl)-3',6'-bis(ethylamino)-2',7'-dimethylspiro[isoindoline-1,9'-xanthen]-3-one;
(c) 2-(2-(((1*H*-pyrrol-2-yl)methyl)amino)ethyl)-3',6'-bis(diethylamino)spiro[isoindoline-1,9'-xanthen]-3-one;
(d) 2-(2-(((1H-pyrrol-2-yl)methyl)amino)ethyl)-3',6'-bis(dimethylamino)spiro[isoindoline-1,9'-xanthen]-3-one;
(e) 2-(2-(((1*H-*pyrrol-2-yl)methyl)amino)ethyl)-3',6'-bis(phenylamino)spiro[isoindoline-1,9'-xanthen]-3-one;
(f) 2-(2-(((1H-pyrrol-2-yl)methyl)amino)ethyl)-3',6'-di(pyrrolidin-1-yl)spiro[isoindoline-1,9'-xanthen]-3-one;
(g) 2-(2-(((1H-pyrrol-2-yl)methyl)amino)ethyl)-3',6'-diamino-2',7'-dimethylspiro[isoindoline-1,9'-xanthen]-3-one;
(h) 2-(2-(((1H-pyrrol-2-yl)methyl)amino)ethyl)-2',7'-dibutyl-3',6'-bis(diethylamino)spiro[isoindoline-1,9'-xanthen]-3-one;
(i) 2-(2-(((1H-pyrrol-2-yl)methyl)amino)ethyl)-3',6'-dimorpholinospiro[isoindoline-1,9'-xanthen]-3-one;
(j) 2-(2-(((1H-pyrrol-2-yl)methyl)amino)ethyl)-3',6'-di(piperidin-1-yl)spiro[isoindoline-1,9'-xanthen]-3-one;
(k) 2-(2-(((1H-pyrrol-2-yl)methyl)amino)ethyl)-1',2',3',4',8',9',10',11'-octahydrospiro[isoindoline-1,6'-pyrano[3,2-g:5,6-g']diquinolin]-3-one;
(l) 2-(2-(((1H-pyrrol-2-yl)methyl)amino)ethyl)-1',2',3',4',10',11',12',13'-octahydrospiro[isoindoline-1,7'-pyrano[2,3-f:6,5-f']diquinolin]-3-one;
(m) 2-(2-(((1H-pyrrol-2-yl)methyl)amino)ethyl)-2',7'-dimethyl-3',6'-di(piperidin-1-yl)spiro[isoindoline-1,9'-xanthen]-3-one; and
(n) 2-(2-(bis((1H-pyrrol-2-yl)methyl)amino)ethyl)-3',6'-bis(diethylamino)spiro[isoindoline-1,9'-xanthen]-3-one.

Yet still another aspect of the invention provides for a method of detecting fluorescence in a biological cell comprising the steps: (a) incubating the biological cell with a compound described above (e.g., a compound of Formula (I) or Formula (II), or preferred or alternative compound embodiments within said Formulas (I) or (II)); (b) shining excitation light to the incubated cell, preferably wherein the shined light has wavelength of at least from 520 nm to 580 nm, alternatively at 543 nm; and (c) detecting light emission from the compound, preferably from 560 nm to 660 nm. In one embodiment, the method further comprises subjecting the biological cell to zinc, alternatively wherein the biological cell is selected from a eukaryotic cell (such as HeLa cells) or a prokaryotic cell (such as a *Streptococcus* genus of bacterium). In yet still another embodiment, the method is conducted with at least one specific compound previously described above or herein. In one embodiment, the pH when detecting light emission from the inventive compound is conducted at a pH greater than 5.5, preferably greater than 6, more preferably greater than 6.0, alternatively from pH 6 to pH 14, alternatively from pH 6 to pH 12, alternatively from pH 6.5 to pH 8.

An alternative aspect of the invention provides for a method of detecting Hg²⁺ in a product composition, comprising the steps: (a) incubating the product composition with a compound of the present invention; (b) shining excitation light to the incubated product composition; (c) detecting light emission from the compound, preferably from 560 nm to 660 nm; wherein preferably the product composition is a personal care product composition, more preferably the personal care product composition is a skin care product composition or a cosmetic product composition. In one embodiment, the product composition for detecting Hg²⁺ is free or substantially free of Zn²⁺.

An alternative aspect of the invention provides for a method of assessing Zn²⁺ chelating effectiveness of Zn²⁺ chelator or a Zn²⁺ chelating system comprising the steps: (a) incubating the Zn²⁺ chelator or a Zn²⁺ chelating system with a compound of the present invention; (b) shining excitation light to the incubated Zn²⁺ chelator or a Zn²⁺ chelating system; and (c) detecting light emission from the compound, preferably from 560 nm to 660 nm. One non-limiting example of a Zn²⁺ chelating system is a dentifrice composition or a mouthwash composition.

### BRIEF DESCRIPTION OF THE DRAWINGS

Figure 1a - 1f are CLSM (Confocal Laser Scanning Microscopy) images of HeLa cells. The data demonstrates that inventive Compound R3 is selective for Zn²⁺ determination in HeLa cells.
Figure 2a - 2f are CLSM images of *Streptococcus* mutans (ATCC® 700610™) cells. The data demonstrates that inventive Compound R3 is selective for Zn²⁺ determination in *Streptococcus* mutans (ATCC® 700610™) cells.

### DETAILED DESCRIPTION OF THE INVENTION

### Definitions:

For purposes of the present invention the term "hydrocarbyl" is defined herein as any organic unit or moiety which is comprised of carbon atoms and hydrogen atoms. Included within the term hydrocarbyl are heterocycles. Non-limiting examples of various unsubstituted non-heterocyclic hydrocarbyl units include pentyl, 3-ethyloctanyl, 1,3-dimethylphenyl, cyclohexyl, cis-3-hexyl, 7, 7-dimethylbicyclo[2.2.1]-heptan-1-yl, and napth-2-yl. Included with the definition of "hydrocarbyl" are the aromatic (aryl) and non-aromatic carbocyclic rings. The term "heterocycle" includes both aromatic (heteroaryl) and non-aromatic heterocyclic rings.

The term "substituted" is used throughout the specification. The term "substituted" is defined herein as "encompassing moieties or units which can replace a hydrogen atom, two hydrogen atoms, or three hydrogen atoms of a hydrocarbyl moiety. Also substituted can include replacement of hydrogen atoms on two adjacent carbons to form a new moiety or unit." For example, a substituted unit that requires a single hydrogen atom replacement includes halogen, hydroxyl, and the like. A two hydrogen atom replacement includes carbonyl, oximino, and the like. A two hydrogen atom replacement from adjacent carbon atoms includes epoxy, and the like. Three hydrogen atom replacement includes cyano, and the like. An epoxide unit is an example of a substituted unit which requires replacement of a hydrogen atom on adjacent carbons. The term "substituted" is used through the present specification to indicate that a hydrocarbyl moiety, *inter alia,* aromatic ring, alkyl chain, can have one or more of the hydrogen atoms replaced by a substituent. When a moiety is described a "substituted" any number of the hydrogen atoms may be replaced. For example, 4-hydroxyphenyl is a "substituted aromatic carbocyclic ring," (N, N-dimethyl-5-amino)octanyl is a "substituted C₈ alkyl unit", 3-guanidinopropyl is a "substituted C₃ alkyl unit," and 2-carboxypyridinyl is a "substituted heteroaryl unit".

In one embodiment, wherein R₁, R₂, R₃, R₄, R₅, R₆, R₇, R₈, R₉, R₁₀, R₁₁, R_{12,} R₁₃, R_{14,} R₁₅, R₁₆ and R₁₇ are each independently selected from the group consisting of H, alkyl, alkenyl, alkynyl, cycloalkyl, cycloalkenyl, aryl, heteroakyl, heteroalkenyl, heteroalkynyl, heterocycloalkyl, heterocycloalkenyl, heteroaryl, substituted, and wherein the aforementioned may be substituted or unsubstituted. These terms are well known in the art. For a detailed description, see U.S. Pat. No. 6,919,346 B2 at column 2, line 61 to column 9, line 53, incorporated herein by reference.

### Synthesis:

Unless otherwise noted, materials are obtained from commercial suppliers and are used without further purification. Rhodamine (95%) is obtained from Sinopharm Chemical Reagent Co., Ltd. (Shanghai). Other chemicals are provided from Shanghai No. 1 chemical reagent. Flash chromatography is carried out on silica gel (200-300 mesh). The ¹H NMR (500 MHz) and ¹³C NMR (125 MHz) spectra are recorded on a Bruker DRX-500 spectrometer. Proton chemical shifts are reported in parts per million downfield from tetramethylsilane (TMS). HRMS is recorded on LTQ-Orbitrap mass spectrometer (ThermoFIsher, San Jose, CA). Melting points are determined on a hot-plate melting point apparatus XT4-100A and uncorrected. UV-Vis spectra is recorded on a Shimadzu UV-2250 spectrophotometer. Fluorescence spectra are recorded on an Edinburgh FLS-920 spectrophotometer. All pH measurements are made with a model Mettler - Toledo meter.

The synthesis of an exemplary compound of the present invention "Compound R3," and an intermediate Compound R5 thereof, is described.

The synthesis of intermediate Compound R5 or simply "R5" is described (2-(2-aminoethyl)-3',6'-bis(diethylamino)spiro[isoindoline-1,9'-xanthen]-3-one). Rhodamine B hydrochloride (5.0 g, 10.4 mmol) and ethylenediamine (12.5 g, 208.8 mmol) are dissolved in ethanol (50 mL) and refluxed for 12 hours. Most of the solvent is removed by evaporation, and the residue is dispersed in water with magnetic stirring. A pink precipitate appears and is recovered by filtration, and thereafter washed thoroughly with water. Lastly, the pink precipitate is washed with petroleum ether, and then dried in vacuum, yielding Compound R5 as a pink powder (3.6 g, 72% yield): ¹H NMR (400 MHz, CDCl₃) δ 7.91 (d, *J =* 2.3 Hz, 1H), 7.45 (d, *J =* 2.5 Hz, 2H), 7.14 - 7.05 (m, 1H), 6.44 (s, 1H), 6.42 (s, 1H), 6.37 (d, *J=* 2.3 Hz, 2H), 6.28 (d, *J=* 2.3 Hz, 1H), 6.26 (d, *J =* 2.4 Hz, 1H), 3.33 (q, *J =* 7.0 Hz, 9H), 3.19 (t, *J =* 6.6 Hz, 2H), 2.40 (t, *J =* 6.6 Hz, 2H), 1.62 (s, 5H), 1.16 (t, *J=* 7.0 Hz, 13H).

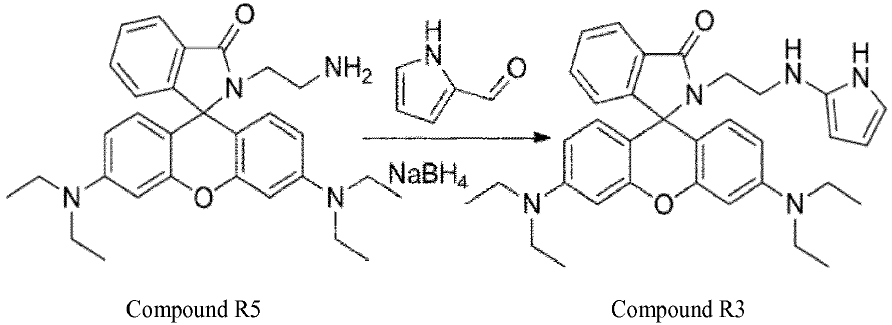

Intermediate Compound R5 (0.2 g, 0.4 mmol) and pyrrole-2-carboxaldehyde (40 mg, 0.4 mmol) are stirred in ethanol at 60°C for 4 hours. The reaction mixture is cooled to 0 °C, and sodium borohydride (1.2 mol equiv) is added. The ice bath is removed 0.5 hour later. The reaction mixture is maintained at room temperature overnight. Evaporating the solvent yields a crude that is purified by column chromatography using ethyl acetate /MeOH(10/1, v/v) to provide inventive Compound R3 (2-(2-(((1H-pyrrol-2-yl)methyl)amino)ethyl)-3',6'-bis(diethylamino)spiro[isoindoline-1,9'-xanthen]-3-one) as a white solid (0.15 g, 67% yield). ¹H NMR (500 MHz, CDCl₃) δ 9.25 (s, 1H), 7.95 - 7.87 (m, 1H), 7.51 - 7.42 (m, 2H), 7.13 - 7.05 (m, 1H), 6.72 (s, 1H), 6.42 (d, *J =* 8.8 Hz, 2H), 6.37 (s, 2H), 6.26 (d, *J =* 8.8 Hz, 2H), 6.06 (s, 1H), 5.92 (s, 1H), 3.65 (s, 2H), 3.33 (dt, *J=* 13.7, 6.7 Hz, 10H), 2.36 (t, *J=* 6.0 Hz, 2H), 1.74 (s, 1H), 1.16 (t, *J=* 6.9 Hz, 12H). ¹³C NMR (126 MHz, CDCl₃) δ 168.88, 153.55, 153.33, 148.86, 132.50, 131.18, 130.50, 128.73, 128.10, 123.85, 122.76, 117.32, 108.17, 107.61, 106.01, 105.51, 97.77, 65.15, 46.88, 45.03, 44.37, 39.34, 12.60. HRMS calc. for C₃₄H₄₂N₅O₂ (M+H⁺): 564.3339, found: 564.3331.

Many further derivations from this basic molecule of inventive Compound R5 can be made by those skilled in the art consistent with Formulas (I) and (II) by using starting materials and intermediates that are known or commercially available or by further modifying these molecules by known methods. Non-limiting examples of these compounds within the scope of Formula (I) and/or Formula (II), and their starting materials, are provided::
(a) 2-(2-(((1*H*-pyrrol-2-yl)methyl)amino)ethyl)-3',6'-diaminospiro[isoindoline-1,9'-xanthen]-3-one: 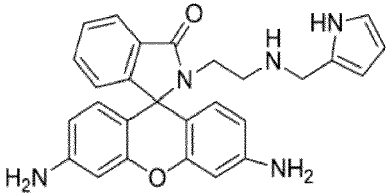 The above compound can be made with starting material: 3',6'-diamino-3H-spiro[isobenzofuran-1,9'-xanthen]-3-one. Referencing formulas (1) and (II), notably R₃, R₄, R₅, R₆ are all each hydrogen. In alternative embodiments, the other variables (R₁ etc.) may be as previously described.
(b) 2-(2-(((1H-pyrrol-2-yl)methyl)amino)ethyl)-3',6'-bis(ethylamino)-2',7'-dimethylspiro[isoindoline-1,9'-xanthen]-3-one: 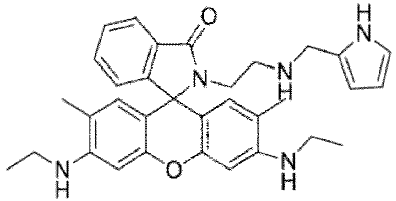 The above compound can be made with starting material: 3',6'-bis(ethylamino)-2',7'-dimethyl-3H-spiro[isobenzofuran-1,9'-xanthen]-3-one. Referencing formulas (I) and (II), notably R₁₄ and R₁₅ are each methyl, R₃ and R₆ are each ethyl, and R₄ and R₅ are each hydrogen. Alternatively, R₁₄ and R₁₅ are each independently selected from C₁-C₁₀ alkyl, substituted or unsubstituted, branched or unbranched; or C₁-C₅ alkyl, unsubstituted and unbranched. Alternatively R₃ and R₆ are each independently selected from C₁-C₁₀ alkyl, substituted or unsubstituted, branched or unbranched; or C₁-C₅ alkyl, unsubstituted and unbranched. In alternative embodiments, the other variables (R₁ etc.) maybe as previously described.
(c) 2-(2-(((1*H*-pyrrol-2-yl)methyl)amino)ethyl)-3',6'-bis(diethylamino)spiro[isoindoline-1,9'-xanthen]-3-one: 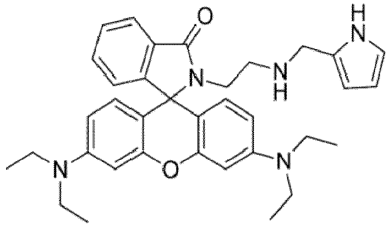 The above compound can be made with starting material: 3',6'-bis(diethylamino)-3H-spiro[isobenzofuran-1,9'-xanthen]-3-one. Referencing formulas (1) and (II), notably R₃, R₄, R₅, and R₆ are each ethyl. Alternatively, R₃, R₄, R₅, and R₆ are each independently selected from C₁-C₁₀ alkyl, substituted or unsubstituted, branched or unbranched; or C₁-C₅ alkyl, unsubstituted and unbranched. In alternative embodiments, the other variables (R₁ etc.) may be as previously described.
(d) 2-(2-(((1H-pyrrol-2-yl)methyl)amino)ethyl)-3',6'-bis(dimethylamino)spiro[isoindoline-1,9'-xanthen]-3-one: 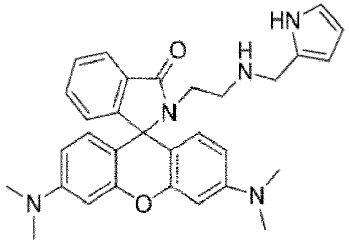 The above compound can be made with starting material: 3',6'-bis(dimethylamino)-3H-spiro[isobenzofuran-1,9'-xanthen]-3-one. Referencing formulas (1) and (II), notably R₃, R₄, R₅, and R₆ are each methyl. Alternatively, R₃, R₄, R₅, and R₆ are each independently selected from C₁-C₁₀ alkyl, substituted or unsubstituted, branched or unbranched; or C₁-C₅ alkyl, unsubstituted and unbranched. In alternative embodiments, the other variables (R₁ etc.) may be as previously described.
(e) 2-(2-(((1*H*-pyrrol-2-yl)methyl)amino)ethyl)-3',6'-bis(phenylamino)spiro[isoindoline-1,9'-xanthen]-3-one: 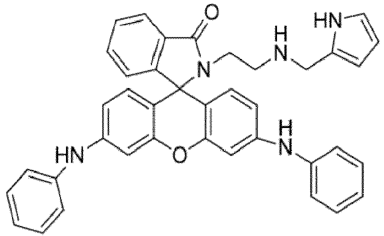 The above compound can be made with starting material: 3',6'-bis(phenylamino)-3H-spiro[isobenzofuran-1,9'-xanthen]-3-one. Referencing formulas (1) and (II), notably R₃ and R₆ are hydrogen while R₄ and R₅ are phenyl. Alternatively R₄ and R₅ are each independently aryl or heteroaryl, substituted or unsubstituted. In alternative embodiments, the other variables (R₁ etc.) may be as previously described.
(f) 2-(2-(((1H-pyrrol-2-yl)methyl)amino)ethyl)-3',6'-di(pyrrolidin-1-yl)spiro[isoindoline-1,9'-xanthen]-3-one: 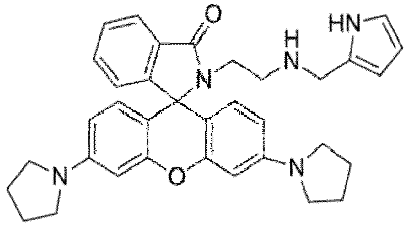 The above compound can be made with starting material: 3',6'-di(pyrrolidin-1-yl)-3H-spiro[isobenzofuran-1,9'-xanthen]-3-one. Referencing formulas (1) and (II), notably R₃ and R₄ together form a pyrrolidinyl moiety, and R₅ and R₆ together form a pyrrolidinyl moiety. Alternatively R₃ and R₄ together form a moiety selected from the group consisting of a cycloalkyl, cycloalkenyl, heterocycloalkyl, and heterocycloalkenyl. Alternatively R₅ and R₆ together form a moiety selected from the group consisting of a cycloalkyl, cycloalkenyl, heterocycloalkyl, and heterocycloalkenyl. In alternative embodiments, the other variables (R₁ etc.) maybe as previously described.
(g) 2-(2-(((1H-pyrrol-2-yl)methyl)amino)ethyl)-3',6'-diamino-2',7'-dimethylspiro[isoindoline-1,9'-xanthen]-3-one: 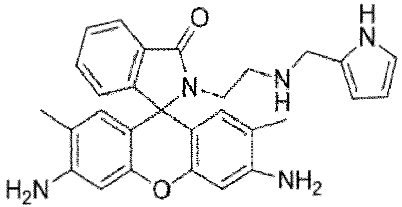 The above compound can be made with starting material: 3',6'-diamino-2',7'-dimethyl-3H-spiro[isobenzofuran-1,9'-xanthen]-3-one. Referencing formula (I) and (II), R₁₄ and R₁₅ are notably methyl. Alternatively R₁₄ and R₁₅ are each independently selected from C₁-C₁₀ alkyl, substituted or unsubstituted, branched or unbranched; or C₁-C₅ alkyl, unsubstituted and unbranched. Referencing formula (1) and (II), R₃, R₄, R₅, R₆ are each notably hydrogen. In alternative embodiments, the other variables (R₁ etc.) maybe as previously described.
(h) 2-(2-(((1H-pyrrol-2-yl)methyl)amino)ethyl)-2',7'-dibutyl-3',6'-bis(diethylamino)spiro[isoindoline-1,9'-xanthen]-3-one: 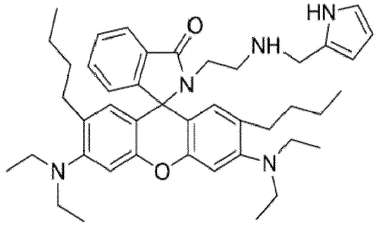 The above compound can be made with starting material: 2',7'-dibutyl-3',6'-bis(diethylamino)-3H-spiro[isobenzofuran-1,9'-xanthen]-3-one. Referencing formula (I) and (II), R₁₄ and R₁₅ are notably methyl. Alternatively R₁₄ and R₁₅ are each independently selected from C₁-C₁₀ alkyl, substituted or unsubstituted, branched or unbranched; or C₁-C₅ alkyl, unsubstituted and unbranched. Referencing formula (1) and (II), R₃, R₄, R₅, and R₆ are each ethyl. Alternatively, R₃, R₄, R₅, and R₆ are each independently selected from C₁-C₁₀ alkyl, substituted or unsubstituted, branched or unbranched; or C₁-C₅ alkyl, unsubstituted and unbranched.
(i) 2-(2-(((1H-pyrrol-2-yl)methyl)amino)ethyl)-3',6'-dimorpholinospiro[isoindoline-1,9'-xanthen]-3-one: 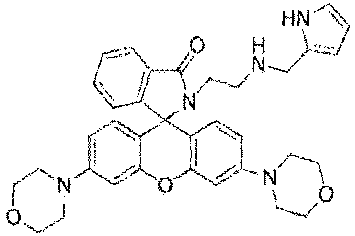 The above compound can be made by starting material: 3',6'-dimorpholino-3H-spiro[isobenzofuran-1,9'-xanthen]-3-one. Referencing formula (I) and (II),
   notably R₃ and R₄ together form a morpholinyl moiety, and R₅ and R₆ together form a morpholinyl moiety. Alternatively R₃ and R₄ together form a moiety selected from the group consisting of a cycloalkyl, cycloalkenyl, heterocycloalkyl, and heterocycloalkenyl. Alternatively R₅ and R₆ together form a moiety selected from the group consisting of a cycloalkyl, cycloalkenyl, heterocycloalkyl, and heterocycloalkenyl. In alternative embodiments, the other variables (R₁ etc.) maybe as previously described.
(j) 2-(2-(((1H-pyrrol-2-yl)methyl)amino)ethyl)-3',6'-di(piperidin-1-yl)spiro[isoindoline-1,9'-xanthen]-3-one: 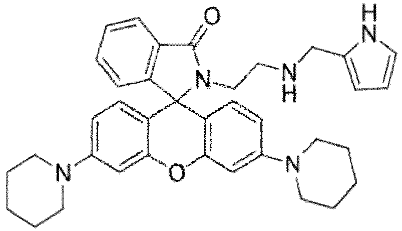 The above compound can be made by starting material: 3',6'-di(piperidin-1-yl)-3H-spiro[isobenzofuran-1,9'-xanthen]-3-one. Referencing formula (I) and (II),
   notably R₃ and R₄ together form a piperidinyl moiety, and R₅ and R₆ together form a piperidinyl moiety. Alternatively R₃ and R₄ together form a moiety selected from the group consisting of a cycloalkyl, cycloalkenyl, heterocycloalkyl, and heterocycloalkenyl. Alternatively R₅ and R₆ together form a moiety selected from the group consisting of a cycloalkyl, cycloalkenyl, heterocycloalkyl, and heterocycloalkenyl. In alternative embodiments, the other variables (R₁ etc.) maybe as previously described.
(k) 2-(2-(((1H-pyrrol-2-yl)methyl)amino)ethyl)-1',2',3',4',8',9',10',11'-octahydrospiro[isoindoline-1,6'-pyrano[3,2-g:5,6-g']diquinolin]-3-one: 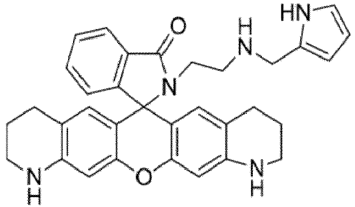 The above compound can be made with starting material: 1',2',3',4',8',9',10',11'-octahydro-3H-spiro[isobenzofuran-1,6'-pyrano[3,2-g:5,6-g']diquinolin]-3-one. Referencing formula (I) and (II), notably R₃ and R₁₄ together form a fused piperidine moiety, and R₆ and R₁₅ together form a fused piperidine moiety. R₄ and R₅ are hydrogen. Alternatively R₃ and R₁₄ together form a fused moiety selected from the group consisting of a cycloalkyl, cycloalkenyl, heterocycloalkyl, and heterocycloalkenyl. Alternatively R₅ and R₁₅ together form a fused moiety selected from the group consisting of a cycloalkyl, cycloalkenyl, heterocycloalkyl, and heterocycloalkenyl. Alternatively R₄ and R₅ are each independently selected from hydrogen, C₁-C₁₀ alkyl, substituted or unsubstituted, branched or unbranched; or C₁-C₅ alkyl, unsubstituted and unbranched. In alternative embodiments, the other variables (R₁ etc.) maybe as previously described.
(l) 2-(2-(((1H-pyrrol-2-yl)methyl)amino)ethyl)-1',2',3',4',10',11',12',13'-octahydrospiro[isoindoline-1,7'-pyrano[2,3-f:6,5-f']diquinolin]-3-one: 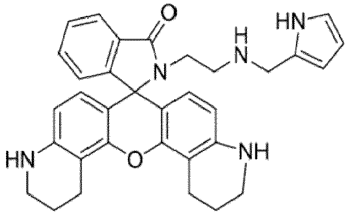 The above compound can be made with starting material: 1',2',3',4',10',11',12',13'-octahydro-3H-spiro[isobenzofuran-1,7'-pyrano[2,3-f:6,5-f']diquinolin]-3-one. Referencing formula (I) and (II), notably R₄ and R₁₆ together form a fused piperidine moiety, and R₅ and R₁₇ together form a fused piperidine moiety. R₃ and R₆ are hydrogen. Alternatively R₄ and R₁₆ together form a fused moiety selected from the group consisting of a cycloalkyl, cycloalkenyl, heterocycloalkyl, and heterocycloalkenyl. Alternatively R₅ and R₁₇ together form a fused moiety selected from the group consisting of a cycloalkyl, cycloalkenyl, heterocycloalkyl, and heterocycloalkenyl. Alternatively R₃ and R₆ are each independently selected from hydrogen, C₁-C₁₀ alkyl, substituted or unsubstituted, branched or unbranched; or C₁-C₅ alkyl, unsubstituted and unbranched. In alternative embodiments, the other variables (R₁ etc.) maybe as previously described.
(m) 2-(2-(((1H-pyrrol-2-yl)methyl)amino)ethyl)-2',7'-dimethyl-3',6'-di(piperidin-1-yl)spiro[isoindoline-1,9'-xanthen]-3-one: 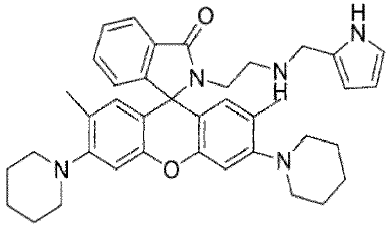 The above compound can be made with starting material: 2',7'-dimethyl-3',6'-di(piperidin-1-yl)-3H-spiro[isobenzofuran-1,9'-xanthen]-3-one. Referencing formula (1) and (II), notably R₃ and R₄ together form a piperidinyl moiety, and R₅ and R₅ together form a piperidinyl moiety. R₁₄ and R₁₅ are each methyl. Alternatively R₃ and R₄ together form a moiety selected from the group consisting of a cycloalkyl, cycloalkenyl, heterocycloalkyl, and heterocycloalkenyl. Alternatively R₅ and R₆ together form a moiety selected from the group consisting of a cycloalkyl, cycloalkenyl, heterocycloalkyl, and heterocycloalkenyl. Alternatively R₁₄ and R₁₅ are each independently selected from C₁-C₁₀ alkyl, substituted or unsubstituted, branched or unbranched; or C₁-C₅ alkyl, unsubstituted and unbranched. In alternative embodiments, the other variables (R₁ etc.) maybe as previously described.
(n) 2-(2-(bis((1H-pyrrol-2-yl)methyl)amino)ethyl)-3',6'-bis(diethylamino)spiro[isoindoline-1,9'-xanthen]-3-one: 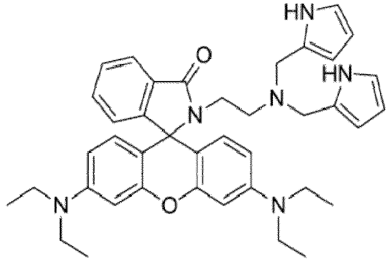 The above compound maybe synthesized according to the following route: 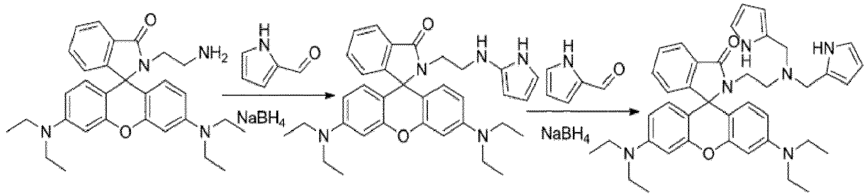 Referencing formula (1) and (II), R₂ is a methylene pyrrol-2-yl. Alternatively R₂ is an alkylene aryl or an alkylene heteroaryl. Alternatively, R₃, R₄, R₅, and R₆ are each independently selected from C₁-C₁₀ alkyl, substituted or unsubstituted, branched or unbranched; or C₁-C₅ alkyl, unsubstituted and unbranched. In alternative embodiments, the other variables (R₁ etc.) may be as previously described.

### Metal Ion Sensing:

The procedure for metal ion sensing is described. Solutions of the metal ions (10.0 mM) are prepared in deionized water. A stock solutions of Compound R3 (0.2 mM) is prepared in ethanol and then diluted to 20 µM with ethanol-water (1: 1, v/v, pH 7.04) for spectral measurement. For titration experiments, a 2.0 mL solution of Compound R3 (20 µM) is filled in a quartz optical cell of 1 cm optical path length. Zn²⁺ stock solution is added into the quartz optical cell gradually by micro-pipette. Spectral data is recorded at 5 min after addition. In selectivity experiments, test samples are prepared by placing appropriate amounts of metal ion stock into 2.0 mL solution of R3 (20 µM). For fluorescence measurements, excitation is provided at 560 nm, while emission is collected from 565 nm to 700 nm.

Results of metal ion response are described. The solution of inventive Compound R3 (20 µM) in control ethanol-water (1: 1, v/v, pH 7.04) is non fluorescent. With addition of Zn²⁺ (0-12 eq), fluorescence at 580 nm is turned on and grew drastically with an excitation of 560 nm. Without wishing to be bound by theory, this is likely due to the ring open reaction of rhodamine induced by Zn²⁺ chelating.

High-level selectivity is of paramount importance for an effective chemosensor. To this end, Compound R3 shows high selectivity on sensing Zn²⁺. Compound R3 (20 µM) in ethanol-water (1: 1, v/v, pH 7.04) demonstrates the greatest fluorescence intensity in presence of Zn²⁺ and Hg²⁺, while other transition and heavy metal ions such as K⁺, Ag⁺, Ca²⁺, Mg²⁺, Pb²⁺, Ni²⁺, Mn²⁺, Co²⁺, Cd²⁺, Cu²⁺, Fe²⁺, Cr³⁺, Li⁺, Ba²⁺, Fe³⁺, Na⁺ display minimal fluorescence intensity at an excitation of 560 nm. Considering that the noxious Hg²⁺ typically does not appear in personal care products and house hold products or biological systems, inventive Compound R3 could be used as a Zn²⁺ sensor.

Table 1 below summarizes florescent spectra data upon addition of different equivalence of Zn²⁺.

**Table 1: Fluorescence spectra of Compound R3 (2.0 x 10⁻⁵ M) upon addition of various metal ions (20.0 x 10⁻⁵M) in ethanol-PBS (1:1, v/v, pH 8.04) with an excitation of 560 nm.**

| Metal Ions: | Peak Fluorescence Intensity (a.u.) at 570-580 nm |
|---|---|
| Hg²⁺ | 1.0 |
| Zn²⁺ | 0.7 |
| Ca²⁺ | 0.25 |
| Others* | < 0.1 |

| | |
|---|---|
| * K⁺, Ag⁺, Ca²⁺, Mg²⁺, Pb²⁺, Ni²⁺, Mn²⁺, Co²⁺, Cd²⁺, Cu²⁺, Fe²⁺, Cr³⁺, Li⁺, Ba²⁺, Fe³⁺, Na⁺ | |

### Methods for detecting mercury

Alternatively, the inventive Compound R3 can be used to study Hg²⁺. One aspect of the invention provides for a method of detecting Hg²⁺ in a product composition, comprising the steps: (a) incubating the product composition with a compound of the present invention; (b) shining excitation light to the incubated product composition; and (c) detecting light emission from the compound from 560 nm to 660 nm;

For example, the approach can be used to detect Hg²⁺ levels in product compositions, including personal care products such as skin care (moisturizers, lotions etc.), hair care (shampoos, conditioners, etc), cosmetic products (make-up, eyeliner, etc.), beauty care products, or other products that are applied to human skin or hair, or the like. Such products typically do not contain Zn²⁺ and thus will not have Zn²⁺ interference thereby making the compounds of the present invention advantageous for such Hg²⁺ detection applications. For example, such a tool could be used to screen for dangerous counterfeit products for the sake of public safety. In some applications, albeit likely less desirable, the compositions of the present invention may be used to screen for Hg²⁺ in digestible products (e.g., food or drink) when Zn²⁺ is not otherwise found (appreciating that trace amounts of Zn²⁺ may interfere with some applications). One skilled in the art will know how to dilute or otherwise prepare the composition for the methods of detecting Hg²⁺ described herein. For example, very viscous composition can be diluted to allow better interface between the probes of the present invention and the subject product compositions containing any Hg²⁺. Solid compositions can be prepared by grinding and extracting any potential Hg²⁺ through solvents and the like before being assessed for Hg²⁺. In one embodiment, the product is a personal care product, more preferably the personal care product is a skin care product or a cosmetic product.

### pH titration

pH titration of Compound R3 is described. Stock solutions of Compound R3 are added to sodium phosphate buffers of various pH to a final concentration of 10 µM. The fluorescence emission spectrum is recorded as a function of pH using *λ*ₑₓ at 560 nm. Titration curves are plotted by fluorescence emission intensities at 580 nm versus pH. Acid-based titration experiment carried out by adjusting the pH with an aqueous solution of NaOH and HCl in phosphate-buffered saline. The titration reveals that the pH range for H⁺ inducing Compound R3 fluorescence is from pH 2.5 to 5.5. Given this rather low pH, the probe is particularly useful in practical applications that are conducted at neutral (or alkaline) pH conditions (such as studying biological cells). For non-H⁺ induced reactions, pH conditions should be greater than 5.5, preferably greater than 6, to mitigate the effect of low pH upon the compound's fluorescence. In one embodiment, the pH conditions in assessing fluorescence of the compounds of the present invention are conducted at pH 6 to 14, preferably pH 6.0 to 12, alternatively from pH 6 to 8.

### Fluorescence imaging of intracellular Zn²⁺

Practical applicability of Compound R3 as a Zn²⁺ probe in the fluorescence imaging of living HeLa cells is demonstrated. Confocal fluorescence imaging is performed with an OLYMPUS IX81 laser scanning microscope and a 60 × oil-immersion objective lens. The microscope is equipped with multiple visible laser lines (405, 488, 543 nm). Images are collected and processed with Olympus FV10-ASW software.

The HeLa cell line is provided by Biochemistry and Cell Biology (China). Cells are grown in DMEM (Dulbecco's modified Eagle's medium) supplemented with 10% FBS (Fetal Bovine serum) and 5% CO₂ at 37°C.

HeLa cells (5 × 10⁻⁸ L⁻¹) are plated on 18 nm glass cover slips and allowed to adhere for 24 hours. 10 µM of Compound R3 in the culture media containing 0.2% (v/v) DMSO is added to the cells. The cells are incubated at 37 °C for 30 min, and washed with PBS three times to remove the excess R3 probe and bathed in PBS (2 mL) before imaging. After washing with PBS (2 mL × 3) to remove the excess probe, the cells are treated with 50 µM Zinc lactate for 30 min. Excitation of Compound R3 loaded cells at 543 nm is carried out with a semiconductor laser, and emission is collected at 560-660 nm (single channel).

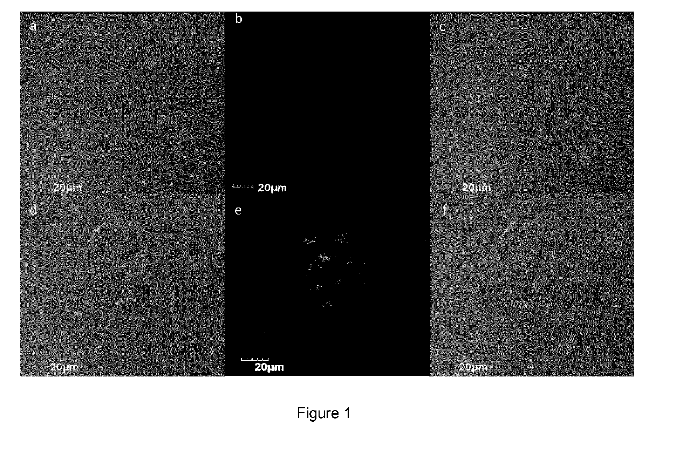

As determined by laser scanning confocal microscopy, Compound R3 gave no fluorescence emission in HeLa cells without Zn²⁺ present (Fig. 1b). When the cells are supplemented with Compound R3 in the PBS for 30 min at 37 °C and then incubated with 50 µM Zn²⁺ under the same conditions, Compound R3 gives a significant fluorescence increase from the intracellular imaging (Fig. 3e). Net, cell imaging experiments indicate that R3 is suitable for detection Zn²⁺ at a cellular level.

Figure 1a - 1f are CLSM (Confocal Laser Scanning Microscopy) images of HeLa cells. Figs. 1a, 1b, and 1c are HeLa cells incubated with 10 µM of inventive Compound R3 for 30 min to assess base level background fluorescence noise. Figs. 1d, 1e, and 1f are cells from the same sample pool subsequently incubated with 50 µM Zn²⁺ for 30 minutes. Red channel emission is collected at 560-660 nm for Fig. 1b and Fig. 1e (*λ*ₑₓ = 543 nm), while Fig. 1a and Fig. 1d are bright field images. Fig. 1c is an overlay of Fig. 1a and Fig. 1b, while Fig. 1f is an overlay of Fig. 1d and Fig. 1e. The data demonstrates that Compound R3 is selective for Zn²⁺ determination in HeLa cells.

### Fluorescence imaging of Zn²⁺ in bacteria

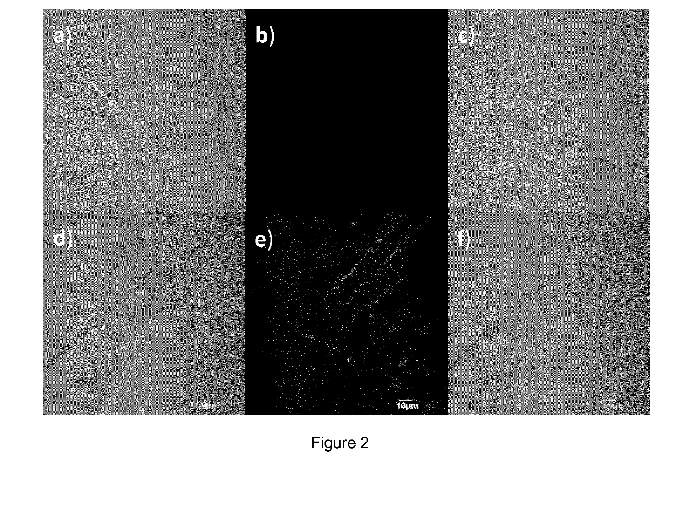

Practical applicability of Compound R3 as Zn²⁺ probe in the fluorescence imaging of living *Streptococcus* mutans (ATCC® 700610™) is demonstrated. The *Streptococcus* mutans is prepared by inoculating the single colony from the BHI agar plate into 5 mL BHI broth and incubating at 37°C for 48 hours. The *Streptococcus* mutans are separately stained with 10 µM of Compound R3 at 37°C for 60 min. As determined by laser scanning confocal microscopy, Compound R3 gives no fluorescence emission without Zn²⁺ present (Fig. 2b). When the *Streptococcus* mutans are supplemented with Compound R3 in the PBS for 60 min at 37°C and then incubated with 50 µM Zn²⁺ under the same conditions, Compound R3 gives a significant fluorescence increase (Fig. 2e). Overlaying fluorescence and bright-field images reveals that the fluorescence signals are localized in the subject *Streptococcus* mutans (Fig. 2f), indicating that Zn²⁺ plays its physiological role within the bacteria.

Figure 2a - 2f are CLSM images of *Streptococcus* mutans (ATCC® 700610™) cells. Figs. 2a, 2b, and 2c are said cells incubated with 10 µM of inventive Compound R3 for 60 min to assess base level background fluorescence noise. Figs. 2d, 2e, and 2f are cells from the same sample pool subsequently incubated with 50 µM Zn²⁺ for 60 min. And then red channel emission is collected at 560-660 nm for Fig. 2b and 2e (*λ*ₑₓ = 543 nm), while Fig. 2a and 2d are bright-field images. Fig. 2c is an overlay of Fig. 2a and Fig. 2b, while Fig. 2f is an overlap of Fig. 2d and Fig. 2e. The data demonstrates that Compound R3 is selective for Zn²⁺ determination in *Streptococcus* mutans (ATCC® 700610™) cells.

In summary, the biological application of inventive Compound R2 is demonstrated by the imaging of Zn²⁺ in HeLa cells and *Streptococcus* mutans (ATCC® 700610™), which is helpful to research of pharmaceutical agents delivery and antibacterial mechanism of Zn²⁺.

### Assessing Zn Chelation Effectiveness

Another application of the composition of the present invention is assessing the effectiveness of a zinc chelator or a zinc chelating system. One aspect of the invention provides a method of assessing the Zn²⁺ chelating effectiveness of Zn²⁺ chelator or a Zn²⁺ chelating system comprising the steps: (a) incubating the Zn²⁺ chelator or a Zn²⁺ chelating system with a compound of the present invention; (b) shining excitation light to the incubated Zn²⁺ chelator or a Zn²⁺ chelating system; and (c) detecting light emission from the compound from 560 nm to 660 nm.

Generally, the more potent the zinc chelator, the less zinc that is available for the Zn²⁺ probe of the present invention to bind and emit less fluorescence. There are at least three variables that may affect zinc chelation. Firstly, the inherent characteristic of any particularly zinc chelator compound. Such compounds include lactate, sulfate, nitrate, chloride, citrate, anions, and the like. Mechanism of actions may include ionic interaction (as in a salt) or covalent binding. Another variable is the concentration of one or more zinc chelators in a zinc chelating system. Generally, the more of a zinc chelator the more zinc chelation is observed. Lastly, other ingredients of the zinc chelating system (e.g., surfactants, binders, other metals, etc.) or physical properties of the system (e.g., pH, viscosity, overall ionic strength, and the like) may influence Zn²⁺ chelation effectiveness. Non-limiting examples of systems may include a dentifrice composition (e.g., toothpaste) or mouthwash, which typically contains zinc salts (such as, for example, for bacterial control or tartar control benefits). Other systems such as shampoo may contain ZPT (Zinc Pyrithione, for antidandruff benefits). The inventive compounds of the present invention may be useful in assessing zinc chelation effectiveness of zinc chelators or zinc chelating system.

Table 2 is the fluorescent relative intensity of inventive Compound R3 (5.0 × 10⁻⁵ M) upon the addition of various zinc compounds. Each sample contains the same level of zinc ion (5.0× 10⁻⁴ M) in H₂O/DMSO (1000:1, V/V) solution, with fluorescence assessed at an excitation of 560 nm.

**Table 2:**

| R3+various Zinc Salts: | Peak Fluorescence Intensity (a.u.) at 570-580 nm: |
|---|---|
| R3+Zinc Lactate | 0.9 |
| R3+Zinc Sulfate | 0.7 |
| R3+Zinc Citrate | 0.3 |
| R3 Only (Control) | 0.1 |

In view of the data, zinc lactate demonstrated the weakest relative degree of zinc chelation effectiveness (among the zinc chelators tested). The findings of Table 2 are consistent with bacterial zinc uptake tests observed in different toothpaste formulations (unpublished). Generally, comparing toothpaste formulations (i.e., zinc chelating systems) containing zinc lactate vs. zinc citrate, those formulations containing zinc lactate are more effective in antibacterial effectiveness which likely attributable to more bioavailable zinc to inhibit bacterial growth or bacterial kill.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of detecting fluorescence in a biological cell comprising the steps:
(a) incubating the biological cell with a zinc fluorescent probe;
(b) shining excitation light to the incubated cell;
(c) detecting light emission from the zinc fluorescent probe from 560 nm to 660 nm; and
**characterized by** subjecting the biological cell to zinc, and the zinc fluorescent probe having the following Formula (I): 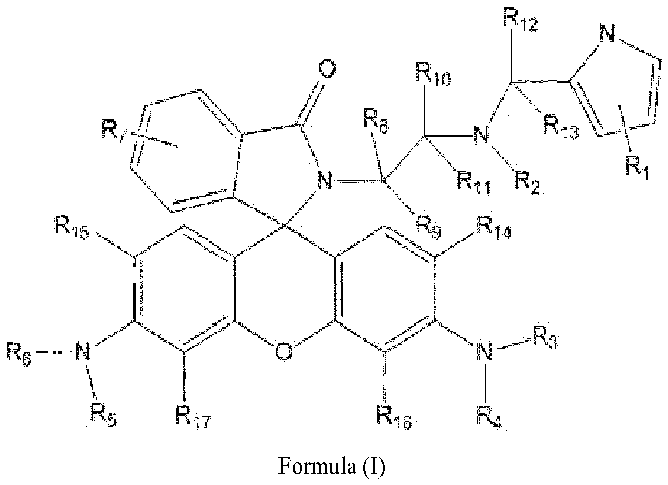 wherein R₁, R₂, R₃, R₄, R₅, R₆, R₇, R₈, R₉, R₁₀, R₁₁, R₁₂, R₁₃, R₁₄, R₁₅, R₁₆, and R₁₇ are each independently a hydrogen or a hydrocarbyl; or an optical isomer, diastereomer or enantiomer of Formula (I), or a salt thereof.

2. The method of claim 1, wherein R₁, R₂, R₃, R₄, R₅, R₆, R₇, R₈, R₉, R₁₀, R₁₁, R₁₂, R₁₃, R₁₄, R₁₅, R₁₆, and R₁₇ are each independently selected from the group consisting of hydrogen, alkyl, alkenyl, alkynyl, cycloalkyl, cycloalkenyl, aryl, heteroakyl, heteroalkenyl, heteroalkynyl, heterocycloalkyl, heterocycloalkenyl, and heteroaryl;
or wherein R₆ and R₁₅; R₅ and R₁₇; or R₅ and R₆; may together form a moiety selected from the group consisting of a cycloalkyl, cycloalkenyl, aryl, heterocycloalkyl, heterocycloalkenyl, and heteroaryl;
or wherein R₄ and R₁₆; R₃ and R₁₄; or R₃ and R₄ may together form a moiety selected from the group consisting of a cycloalkyl, cycloalkenyl, aryl, heterocycloalkyl, heterocycloalkenyl, and heteroaryl; and
wherein any one of the aforementioned may be substituted or unsubstituted.

3. The method of claim 2, wherein: R₁, R₇, R₈, R₉, R₁₀, R₁₁, R₁₂, and R₁₃ are each independently selected from hydrogen, C₁-C₁₀ alkyl or alkenyl, and wherein the aforementioned may be substituted or unsubstituted.

4. The method of claim 3, wherein R₁, R₇, R₈, R₉, R₁₀, R₁₁, R₁₂, and R₁₃ are each independently selected from hydrogen, or unsubstituted C₁-C₅ alkyl.

5. A method of detecting fluorescence in a biological cell comprising the steps:
(a) incubating the biological cell with a zinc fluorescent probe;
(b) shining excitation light to the incubated cell;
(c) detecting light emission from the zinc fluorescent probe from 560 nm to 660 nm; and
**characterized by** subjecting the biological cell to zinc, and the zinc fluorescent probe having the following Formula (II): 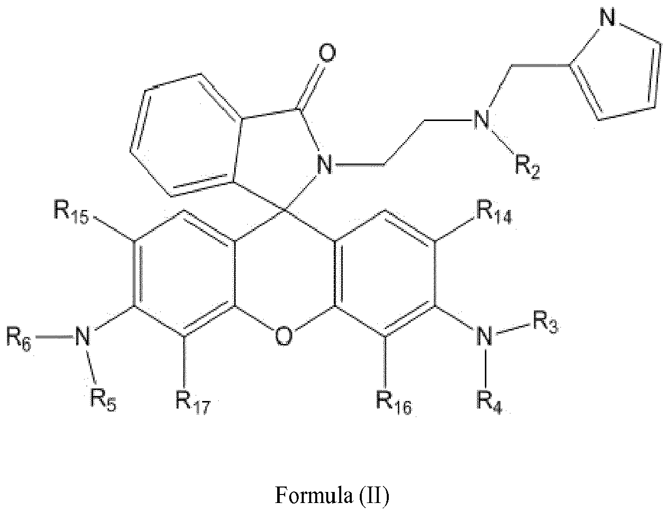 wherein R₂, R₃, R₄, R₅, R₆, R₁₄, R₁₅, R₁₆, and R₁₇ are each independently selected from the group consisting of hydrogen, alkyl, alkenyl, alkynyl, cycloalkyl, cycloalkenyl, aryl, heteroakyl, heteroalkenyl, heteroalkynyl, heterocycloalkyl, heterocycloalkenyl, and heteroaryl;
or wherein R₆ and R₁₅; R₅ and R₁₇; or R₅ and R₆; may together form a moiety selected from the group consisting of a cycloalkyl, cycloalkenyl, aryl, heterocycloalkyl, heterocycloalkenyl, and heteroaryl;
or wherein R₄ and R₁₆; R₃ and R₁₄; or R₃ and R₄ may together form a moiety selected from the group consisting of a cycloalkyl, cycloalkenyl, aryl, heterocycloalkyl, heterocycloalkenyl, and heteroaryl; and
wherein any one of the aforementioned may be substituted or unsubstituted.

6. The method of claim 5, wherein R₂ is hydrogen.

7. The method of claim 6, wherein R₁₄ and R₁₅ are each independently selected from hydrogen, or an unsubstituted, branched or unbranched C₁-C₅ alkyl.

8. The method of claim 6, wherein R₁₆ and R₁₇ are each independently selected from hydrogen, or an unsubstituted, branched or unbranched C₁-C₅ alkyl.

9. The method of claim 5, wherein the zinc fluorescent probe and is selected from the group consisting of:
(a) 2-(2-(((1*H-*pyrrol-2-yl)methyl)amino)ethyl)-3',6'-diaminospiro[isoindoline-1,9'-xanthen]-3-one;
(b) 2-(2-(((1H-pyrrol-2-yl)methyl)amino)ethyl)-3',6'-bis(ethylamino)-2',7'-dimethylspiro[isoindoline-1,9'-xanthen]-3-one;
(c) 2-(2-(((1*H*-pyrrol-2-yl)methyl)amino)ethyl)-3',6'-bis(diethylamino)spiro[isoindoline-1,9'-xanthen]-3-one;
(d) 2-(2-(((1H-pyrrol-2-yl)methyl)amino)ethyl)-3',6'-bis(dimethylamino)spiro[isoindoline-1,9'-xanthen]-3-one;
(e) 2-(2-(((1*H-*pyrrol-2-yl)methyl)amino)ethyl)-3',6'-bis(phenylamino)spiro[isoindoline-1,9'-xanthen]-3-one;
(f) 2-(2-(((1H-pyrrol-2-yl)methyl)amino)ethyl)-3',6'-di(pyrrolidin-1-yl)spiro[isoindoline-1,9'-xanthen]-3-one;
(g) 2-(2-(((1H-pyrrol-2-yl)methyl)amino)ethyl)-3',6'-diamino-2',7'-dimethylspiro[isoindoline-1,9'-xanthen]-3-one;
(h) 2-(2-(((1H-pyrrol-2-yl)methyl)amino)ethyl)-2',7'-dibutyl-3',6'-bis(diethylamino)spiro[isoindoline-1,9'-xanthen]-3-one;
(i) 2-(2-(((1H-pyrrol-2-yl)methyl)amino)ethyl)-3',6'-dimorpholinospiro[isoindoline-1,9'-xanthen]-3-one;
(j) 2-(2-(((1H-pyrrol-2-yl)methyl)amino)ethyl)-3',6'-di(piperidin-1-yl)spiro[isoindoline-1,9'-xanthen]-3-one;
(k) 2-(2-(((1H-pyrrol-2-yl)methyl)amino)ethyl)-1',2',3',4',8',9',10',11'-octahydrospiro[isoindoline-1,6'-pyrano[3,2-g:5,6-g']diquinolin]-3-one;
(l) 2-(2-(((1H-pyrrol-2-yl)methyl)amino)ethyl)-1',2',3',4',10',11',12',13'-octahydrospiro[isoindoline-1,7'-pyrano[2,3-f:6,5-f]diquinolin]-3-one;
(m) 2-(2-(((1H-pyrrol-2-yl)methyl)amino)ethyl)-2',7'-dimethyl-3',6'-di(piperidin-1-yl)spiro[isoindoline-1,9'-xanthen]-3-one; and
(n) 2-(2-(bis((1H-pyrrol-2-yl)methyl)amino)ethyl)-3',6'-bis(diethylamino)spiro[isoindoline-1,9'-xanthen]-3-one.

10. The method of any one of the proceeding claims, when a pH from a solution containing the zinc fluorescent probe which light emission is detected, is greater than pH 5.5, preferably greater than 6, more preferably greater than 6.0.

11. The method of any one of the proceeding claims, wherein the biological cell is selected from a HeLa cell or a *Streptococcus* genus of bacterium.

12. A method of detecting Hg²⁺ in a product composition comprising the steps:
(a) incubating the product composition with a zinc fluorescent probe;
(b) shining excitation light to the incubated product composition; and
(c) detecting light emission from the zinc fluorescent probe from 560 nm to 660 nm; and **characterized by** the zinc fluorescent probe having the following Formula (I): 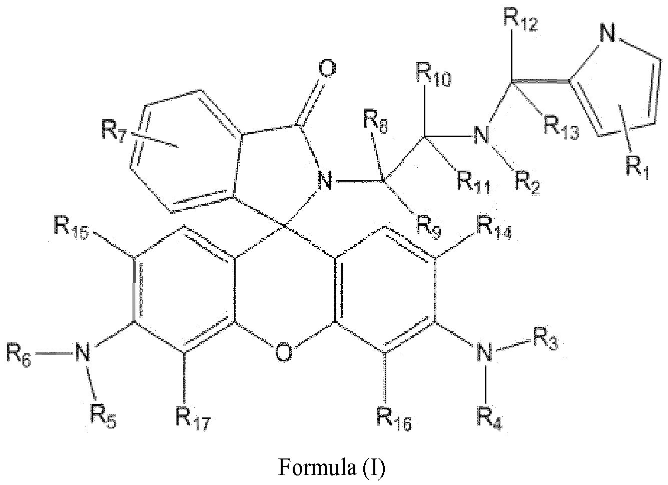 wherein R₁, R₂, R₃, R₄, R₅, R₆, R₇, R₈, R₉, R₁₀, R₁₁, R₁₂, R₁₃, R₁₄, R₁₅, R₁₆, and R₁₇ are each independently a hydrogen or a hydrocarbyl; or an optical isomer, diastereomer or enantiomer of Formula (I), or a salt thereof.

13. The method of claim 12, wherein the product composition is a personal care product composition or a cosmetic care product composition.

14. A method of assessing the Zn²⁺ chelating effectiveness of Zn²⁺ chelator or a Zn²⁺ chelating system comprising the steps:
(a) incubating the Zn²⁺ chelator or a Zn²⁺ chelating system with a zinc fluorescent probe;
(b) shining excitation light to the incubated Zn²⁺ chelator or a Zn²⁺ chelating system;
(c) detecting light emission from the zinc fluorescent probe from 560 nm to 660 nm.
**characterized by** the zinc fluorescent probe having the following Formula (I): 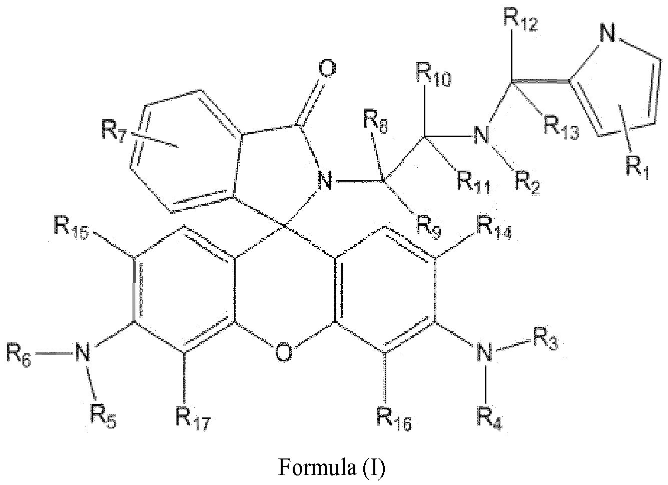 wherein R₁, R₂, R₃, R₄, R₅, R₆, R₇, R₈, R₉, R₁₀, R₁₁, R₁₂, R₁₃, R₁₄, R₁₅, R₁₆, and R₁₇ are each independently a hydrogen or a hydrocarbyl; or an optical isomer, diastereomer or enantiomer of Formula (I), or a salt thereof.

15. The method of claim 14, wherein a pH from a solution containing the zinc fluorescent probe which light emission is detected, is greater than pH 5.5.
